Europäisches Patentamt

**European Patent Office**

Office européen des brevets

(11) Publication number : **0 495 675 A1**

(12) # EUROPEAN PATENT APPLICATION

(21) Application number : **92300431.1**

(22) Date of filing : **17.01.92**

(51) Int. Cl.⁵ : **A61M 15/00**

(30) Priority : **18.01.91 GB 9101174**

(43) Date of publication of application :
**22.07.92 Bulletin 92/30**

(84) Designated Contracting States :
**PT**

(71) Applicant : **FISONS plc**
**Fison House Princes Street**
**Ipswich Suffolk IP1 1QH (GB)**

(72) Inventor : **Hart, John Leck**
**74 Cow Lane**
**Bramcote, Nottingham NG9 3BB (GB)**

(74) Representative : **Wright, Robert Gordon McRae**
**FISONS plc 12 Derby Road**
**Loughborough Leicestershire LE11 0BB (GB)**

(54) **Inhalation devices.**

(57)    A device for the administration of an inhalation medicament, comprises a body 1,2 defining a through-going pathway terminating in a mouthpiece 4 and means for dispensing inhalation medicament into the pathway 7,8,9, characterised in that the device includes a flavour modifying agent 12 separated from the inhalation medicament, such that the medicament and the flavour modifying agent do not interact prior to dispensation of the medicament into the through-going pathway.

Fig.1.

EP 0 495 675 A1

This invention relates to devices for administration of medicaments by inhalation, particularly medicaments having an unpleasant taste or odour.

Medicaments for inhalation are commonly administered in the form of powders, either from pressurised canisters containing the powder in admixture with a liquefied gas aerosol propellant, or as a dry powder, e.g. from the device known by the registered trademark SPINHALER.

In the former case it is known to include in the formulation flavouring and/or sweetening agents to improve the taste of the formulation and/or to give the formulation a recognisable flavour so that the patient realises when a dose has been inhaled, as disclosed in EP-A-0365119. However, a good deal of formulation work and stability testing must be carried out in order to produce a satisfactory formulation.

Whilst sweeteners may be incorporated into dry powder formulations, flavouring agents (which are typically volatile oils) cannot, for example, because they have poor flow properties.

We now provide a device for the administration of an inhalation medicament which overcomes these disadvantages.

According to the invention there is provided a device for the administration of an inhalation medicament, comprising a body defining a through-going pathway terminating in a mouthpiece and means for dispensing inhalation medicament into the pathway, characterised in that the device includes a flavour modifying agent separated from the inhalation medicament, such that the medicament and the flavour modifying agent do not interact prior to dispensation of the medicament into the through-going pathway.

Flavour modifying agents include any agent which alters the perceived flavour of the dosage inhaled from the device by the patient. The agent may add flavours and/or odours to, or remove flavours and/or odours from, the air space within the device. The flavour modifying agent can therefore be a volatile flavouring agent or a deodorising agent.

Volatile flavouring agents include any flavouring agent which has a vapour pressure sufficiently high for a detectable and sufficient quantity of the flavouring agent to be entrained in the flow of air through the device and inhaled each time the device is used.

Flavouring agents which may be used include peppermint oil and menthol. The proprietary product known by the tradename Dentomint, which contains both menthol and peppermint oil, may also be used.

Deodorising agents include any agent capable of removing odorous substances from the air space within the device and converting them, for example by absorption, to odourless substances.

One particular deodorising agent which may be mentioned is activated charcoal.

The flavour modifying agent may contain both a volatile flavouring agent and a deodorising agent.

The flavour modifying agent may conveniently be incorporated into a body located within the device, for example, it may be impregnated on a porous body. The body may be located at any convenient position within the device, either upstream or downstream of the point in the pathway at which medicament is dispensed.

In the case of a volatile flavouring agent air flowing over or through the body conveys the aroma to the patient during the course of inhalation of the medicament. In the case of a deodorising agent odorous substances contained within the air space of the device will be absorbed by the body. The latter case may be of particular benefit when the device is stored for long periods of time between doses thus allowing unpleasant odours to build up within the stagnant air space of the device.

The body containing the flavour modifying agent may conveniently be removed from the inhalation device thus allowing its replacement once the action of the existing flavour modifying agent is exhausted. Hence, replacement bodies may be supplied separately from the inhalation device.

Thus, according to a further aspect of the invention, we provide a body comprising a flavour modifying agent, characterised in that the body is adapted for location within a device for the administration of an inhalation medicament.

Inhalation devices for use in accordance with the invention include any device conventionally used for dispensing orally or nasally inhaled medicaments. Suitable devices include multiple dose inhalers (MDI's) used in conjunction with a pressurised aerosol canister of medicament; dry powder inhalers, e.g. that known by the registered trademark SPINHALER; nebulisers, e.g. that known by the registered trademark FISONEB; and spacer devices for use in conjunction with an MDI, e.g. that known by the registered trademark FISONAIR as disclosed in International Patent Application No. PCT/GB 90/01036.

Medicaments for use in accordance with the invention include any medicaments which are conventionally administered by inhalation. Such medicaments include drugs for use in the prophylactic or remedial treatment of reversible obstructive airways disease. Specific medicaments which may be mentioned include, for example, sodium cromoglycate and nedocromil sodium; inhaled steroids such as beclomethasone dipropionate, tipredane, and fluticasone; anticholinergic agents such as ipratropium bromide; bronchodilators, e.g. salmeterol, salbutamol, reproterol, terbutaline and fenoterol; and salts thereof. The invention may be particularly useful for medicaments which have unpleasant tastes and/or aromas, or which have no discernable taste of their own.

The medicament will generally be administered as a composition including one or more additional pharmaceutically acceptable additives. The type of composition will, of course, depend upon the nature of the inhalation device:

For administration by multiple dose inhaler suitable formulations include pressurised powder compositions, e.g. wherein the medicament in finely divided form is in admixture with a pressurised pharmaceutically acceptable propellant.

For administration by dry powder inhaler suitable formulations include non-pressurised powder compositions, e.g. wherein the medicament in finely divided form is in admixture with a pharmaceutically acceptable carrier of larger particle size.

For administration by nebuliser suitable formulations include solutions or dispersions, e.g. aqueous solutions or dispersions.

The device according to the invention is advantageous in that it permits the deployment of a flavour modifying agent in conjunction with an inhalation medicament, without the need to develop a new formulation. The flavour modifying agents are useful in that they may mask or otherwise improve the taste of otherwise unpleasant-tasting medicaments, or they may give the formulation a recognisable flavour so that the patient realises when a dose has been inhaled, or they may absorb unpleasant odours which develop within the device during storage between doses.

A preferred embodiment of the invention will now be described, by way of illustration only, with reference to the accompanying drawings, in which:

Figure 1 is a side view in cross-section of a simple dry powder inhalation device according to the invention containing a perforated capsule of medicament to be inhaled,

Figure 2 is a sectional view on the line II-II′ in Figure 1,

Figure 3 is a sectional view on the line III-III′ in Figure 1,

Figure 4 is side view in cross-section of a simple multiple dose inhalation device according to the invention containing a pressurised canister of medicament to be inhaled,

Figure 5 is a view in partial section along the line IV-IV′ in Figure 4, and

Figure 6 is a perspective view of a body containing a flavour modifying agent suitable for location within the device of Figure 4.

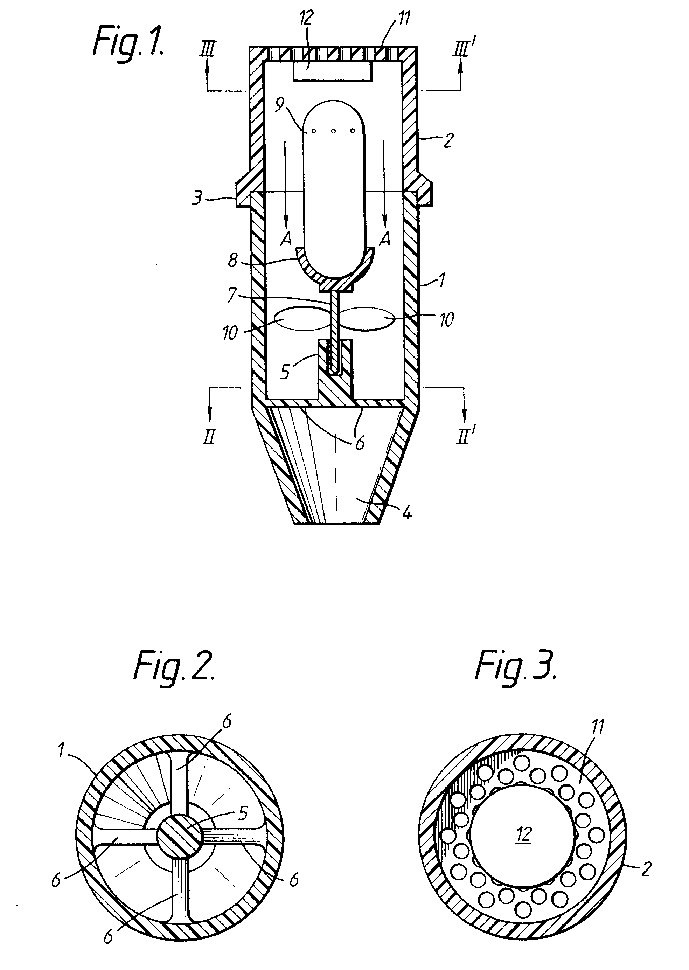

Referring first to Figure 1, a dry powder inhalation device comprises a generally cylindrical housing having two portions, a mouthpiece portion 1 and a closure portion 2 which describe a through-going pathway. Closure portion 2 is provided, at its end which connects with mouthpiece portion 1, with a peripheral flange 3 within which the end of mouthpiece portion 1 fits closely. At its end remote from closure portion 2, mouthpiece portion 1 is tapered to form a frustoconical mouthpiece 4.

Within mouthpiece portion 1 a simple bearing 5 is (as is most clearly seen from Figure 2) supported by cross members 6. A spindle 7 is seated in bearing 5. Spindle 7 is provided with a cup 8 which is capable of closely receiving a perforated capsule 9 containing medicament to be inhaled, which together form means for dispensing the medicament. Spindle 7 is also provided with rotor vanes 10 which cause spindle 7 to rotate within bearing 5 when air is drawn through the device, as during inhalation.

Closure portion 2 is provided at its end remote from mouthpiece portion 1 with a perforated grid 11 (see Figure 3) to which is affixed a flavour modifying agent in the form of a porous body 12 impregnated with a volatile aromatic oil.

In use, mouthpiece portion 1 and closure portion 2 are separated, previously perforated capsule 9 containing the medicament to be inhaled is located in cup 8, and portions 1 and 2 reassembled. The patient inhales through mouthpiece 4, thereby drawing air through the device in the direction of the arrows A in Figure 1. The flow of air through the device causes spindle 7 to rotate. Medicament is thereby dispensed from capsule 9, entrained in the air flow and inhaled. Air drawn into the device through the perforations of grid 11 passes around and through impregnated body 12 and conveys the aroma of the oil to the patient.

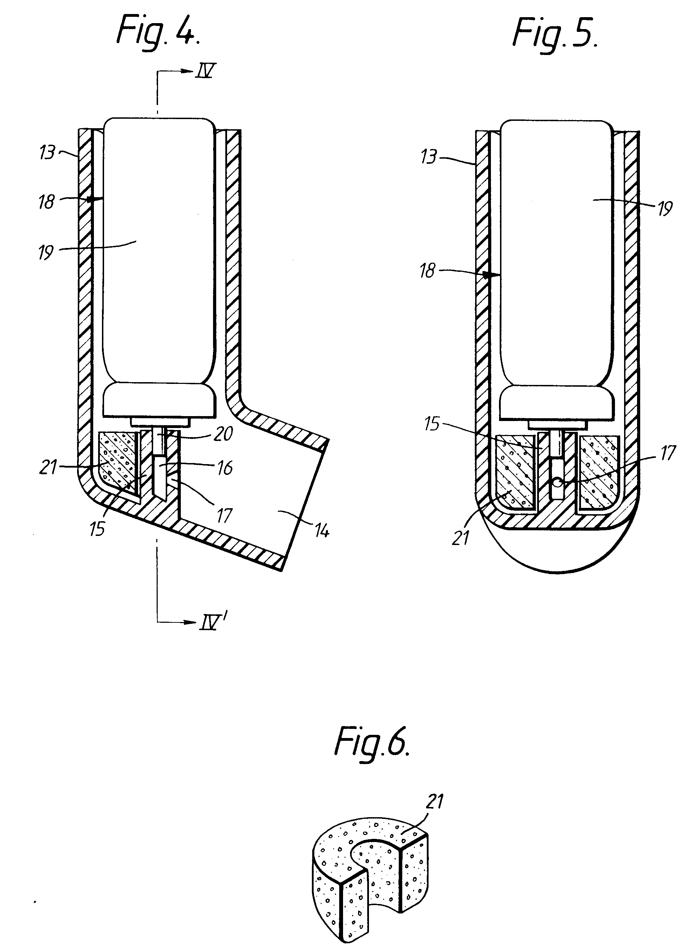

Referring now to Figure 4, the multi-dose inhalation device comprises a through-going pathway in the form of a generally cylindrical housing 13 having a mouthpiece 14 provided at its lower end. Within the mouthpiece end of the housing there is provided a spray head 15 containing an internal cavity 16 and outlet orifice 17.

The housing 13 is adapted to receive means for dispensing an inhalation medicament, comprising a pressurised medicament container 18, having a generally cylindrical body 19 provided at one end with a metering valve including a valve stem 20 adapted to engage the spray head 15. When the container 18 is received into the housing 13 and the valve stem 20 engaged with the spray head 15, a flavour modifying agent, in the form of a porous body 21 (see Figure 6) impregnated with a volatile aromatic oil, is retained at the mouthpiece end of the housing 13, (as is most clearly seen in Figure 5).

In use the patient depresses medicament container 18 towards the spray head 15 thereby dispensing an aerosolised dose of medicament. The patient's breathing is coordinated so as to inhale the medicament as it is dispensed, air drawn into the device passes around and through impregnated body 21 and conveys the aroma of the oil to the patient.

## Claims

1. A device for the administration of an inhalation medicament, comprising a body defining a through-going pathway terminating in a mouthpiece and means for dispensing inhalation medicament into the pathway, characterised in that the device includes a flavour modifying agent separated from the inhalation medicament, such that the medicament and the flavour modifying agent do not interact prior to dispensation of the medicament into the through-going pathway.

2. A device according to claim 1, wherein the flavour modifying agent is impregnated on a porous body located within the device.

3. A device according to claim 1 or 2, wherein the flavour modifying agent is a volatile flavouring agent.

4. A device according to claim 3, wherein the volatile flavouring agent contains peppermint oil and/or menthol.

5. A device according to claim 1 or 2, wherein the flavour modifying agent is a deodorising agent.

6. A device according to claim 5, wherein the deodorising agent is charcoal.

7. A device according to any one of the previous claims, wherein the flavour modifying agent is located upstream of the point in the pathway at which medicament is dispensed.

8. A device according to any one of the preceding claims, wherein the medicament is a pressurised powder composition.

9. A device according to any one of the preceding claims, wherein the medicament is a non-pressurised powder composition.

10. A body comprising a flavour modifying agent, characterised in that the body is adapted for location within a device for the administration of an inhalation medicament.

Fig.1.

Fig.2.

Fig.3.

# Fig. 4.

# Fig. 5.

# Fig. 6.

**European Patent Office**

# EUROPEAN SEARCH REPORT

Application Number

EP 92 30 0431

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-819 074 (W WALKER LTD)<br>* the whole document *<br>--- | 1-3 | A61M15/00 |
| X | DE-A-2 934 245 (H TROST)<br>* the whole document *<br>--- | 1 | |
| X | GB-A-259 361 (M FECHER)<br>* page 2, line 7 - line 41; figures *<br>--- | 1-4,7 | |
| X | FR-A-2 232 333 (NIEDDU)<br>* the whole document *<br><br>----- | 1 | |
| | | | **TECHNICAL FIELDS SEARCHED (Int. Cl.5 )** |
| | | | A61M |

The present search report has been drawn up for all claims

| Place of search | Date of completion of the search | Examiner |
|---|---|---|
| THE HAGUE | 30 MARCH 1992 | VEREECKE A. |

EPO FORM 1503 03.82 (P0401)